# EUROPEAN PATENT APPLICATION

(11) **EP 0 841 047 A1**
(43) Date of publication of application: **13.05.1998**
(21) Application number: 96118135.1
(22) Date of filing: 12.11.1996
(51) Int. Cl.: A61F 9/00, A61H 9/00

(54) **Vision correcting device**

(71) Applicant: Jong Kang Enterprise Co., Ltd., Taichung City (TW); Liu, Hsing Chuan, Taichung City (TW)
(72) Inventor: Liu, Hsing Chuan, Taichung City, (TW)
(74) Representative: Grünecker, Kinkeldey, Stockmair & Schwanhäusser Anwaltssozietät

(57) **Abstract**

A vision correcting device comprises an eye mask, an action apparatus, and an air tube connecting the eye mask with the action apparatus. The action apparatus is automated and is composed of a rotary wheel driven by a built-in motor, a connection rod fastened pivotally with the rotary wheel, and an air cell, when the rotary wheel is driven by the motor to rotate clockwise, the air cell is acted on by the connection rod such that air pressure in the eye mask is increase rapidly for treating myopia. When the rotary wheel is driven by the motor to rotate counterclockwise, the air cell is acted on by the connection rod such that air pressure in the eye mask is decreased so as to bring about a negative pressure in the eye mask for treating hyperopia.

## Description

### FIELD OF THE INVENTION

The present invention relates generally to an ophthalmological device, and more particularly to a vision correcting device.

### BACKGROUND OF THE INVENTION

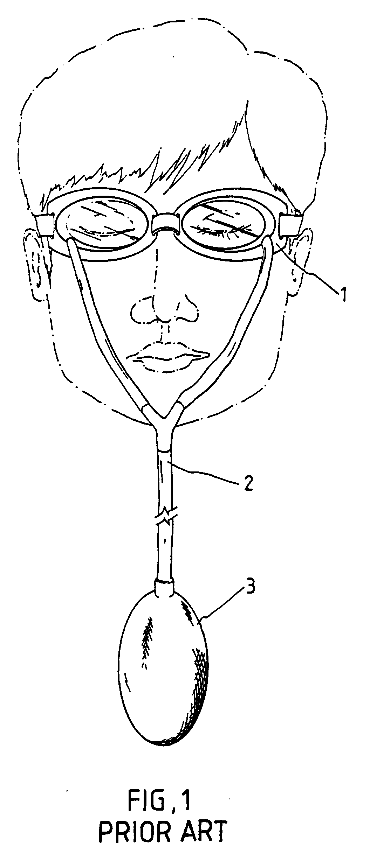

As shown in FIG. 1, a vision correcting device of the prior art comprises an eye mask 1, an air tube 2, and an action device 3 of a spherical construction. The action device 3 is in fact an air sac capable of generating a positive air pressure or a negative air pressure in the eye mask 1. The positive air pressure is generated in the eye mask 1 when the action device 3 is exerted on by an external force. On the other hand, the negative air pressure is generated in the eye mask 1 when the action device 3 is relieved of the external force exerting thereon. The action device 3 is operated with a person's hand and is therefore not precise at best. The vision correcting device of the prior art works in such a manner that a rapid increase in the air pressure in the eye mask 1 can bring about the movement of the eye ball towards the inside of the eye socket. As the treatment is performed for a prolonged period of time, the distance between the front side and the rear side of the eye ball is shortened, thereby resulting in a decrease in the curvature of the eye lens. As a result, the vision correcting device of the prior art is capable of treating the myopia. In the process of treating the hyperopia with the prior art device, the action device 3 is operated such that a rapid decrease in air pressure inside the eye mask 1 is brought about to generate a negative pressure in the eye mask 1, and that the eye ball is gradually caused to protrude toward the front side of the eye socket so as to result in a gradual increase in the distance between the front end and the rear end of the eye ball, thereby bringing about an increase in the curvature of the eye lens.

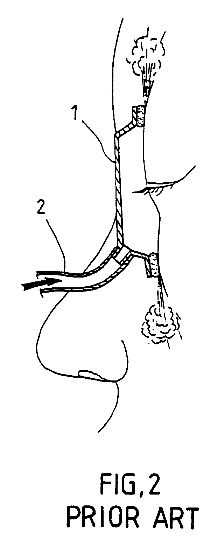

Such a vision correcting device as described above is defective in design in that the action device 3 can not be operated with hand in a precise manner, and that the increase or decrease in the air pressure inside the eye mask 1 can not be therefore brought about with ease and precision, and further that it is difficult for a child to use it correctly, and still further that the eye mask 1 is vulnerable to air leak, as illustrated in FIG. 2.

### SUMMARY OF THE INVENTION

It is thereofre the primary objective of the present invention to provide a vision correcting device free from the short comings of the prior art device described above.

In keeping with the principle of the present invention, the foregoing objective of the present invention is attained by an improved device for correcting myopia and hyperopia. The device of the present invention comprises an eye mask, an air tube, and an action device. The action device is automated and is composed of a rotary wheel driven by a built-in motor, a connection rod fastened pivotally with the rotary wheel, and an air cell. When the rotary wheel is driven by the motor to rotate clockwise, the air cell is acted on by the connection rod such that the air pressure in the eye mask is increased rapidly for treating myopia. When the rotary wheel is driven by the motor to rotate counterclockwise, the air cell is acted on by the connection rod such that the air pressure in the eye mask is decreased so as to bring about a negative pressure in the eye mask for treating hyperopia.

### BRIEF DESCRIPTION OF THE DRAWINGS

FIG. 1 shows a perspective view of a prior art vision correcting device at work.

FIG. 2 shows a partial sectional view of the prior art vision correcting device as shown in FIG. 1.

FIG. 3 shows a perspective view of a vision correcting device of the present invention.

FIG. 4 shows an exploded view of an eye mask of the vision correcting device of the present invention.

FIG. 5 shows a plan sectional view of an automated action device of the vision correcting device of the present invention.

FIG. 6 shows a schematic view of a rotary wheel in action according to the vision correcting device of the present invention.

FIG. 7 shows another schematic view of the rotary wheel in action according to the vision correction device of the present invention.

FIG. 8 shows a plan sectional view of the eye mask at work according to the present invention.

FIG. 9 shows a plan schematic view of the air tube of the vision correcting device of the present invention.

FIG. 10 shows a plan schematic view of an action device of a second preferred embodiment of the present invention.

FIG. 11 shows a plan schematic view of an action device of a third preferred embodiment of the present invention.

FIG. 12 shows a plan schematic view of an action device of a fourth preferred embodiment of the present invention.

FIG. 13 shows a plan schematic view of an action device of a fifth preferred embodiment of the present invention.

FIG. 14 shows a partial sectional view of the present invention.

FIG. 15 shows a schematic view of an eye mask embodied in the present invention.

### DETAILED DESCRIPTION OF THE EMBODIMENTS

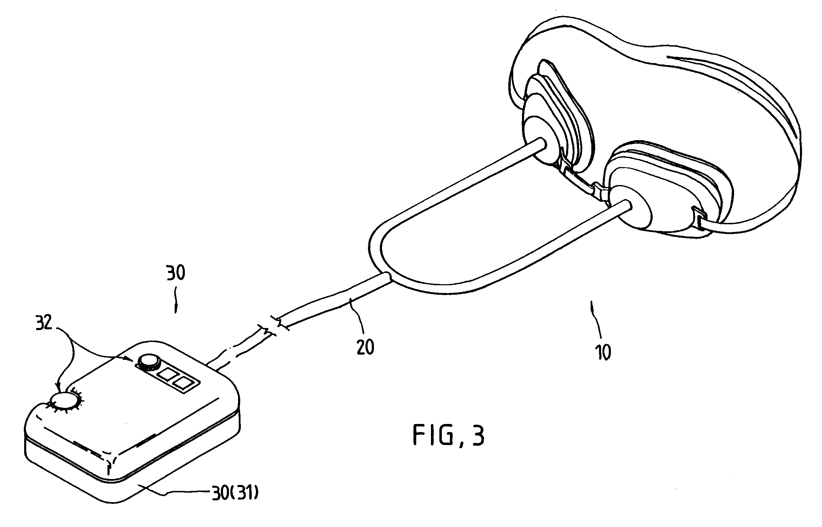

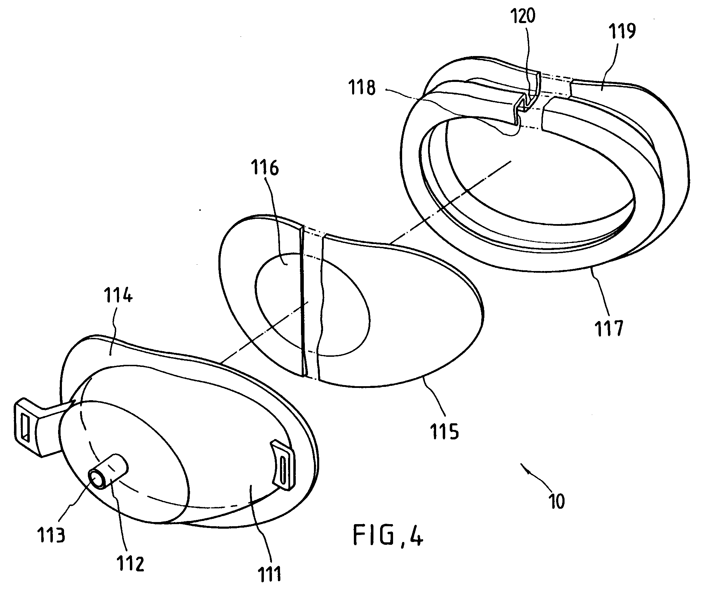

As shown in FIGS. 3 and 4, a vision correcting device embodied in the present invention is composed of an eye mask 10, an air tube 20, and an automated action apparatus 30.

The eye mask 10 is composed of two mask pieces 111 opposite to each other. Each mask piece 111 comprises a pressure piece 115 and a frame 117. The mask piece 111 is provided on the outer surface thereof with a projection 112 having a through hole 113 and is further provided with a rim 114. The pressure piece 115 is thin and similar in profile to the rim 114 and is provided with a buffer piece 116. The frame 117 is provided with a groove 118 and a face frame 119 which is separated from the groove 118 by an appopriate interval 120. The groove 118 is intended to receive therein the rim 114. The pressure piece 115 is held between the mask piece 111 and the frame 117.

The air tube 20 is fastened between the eye mask 10 and the action apparatus 30 such that the eye mask 10 is in communication with the action apparatus 30.

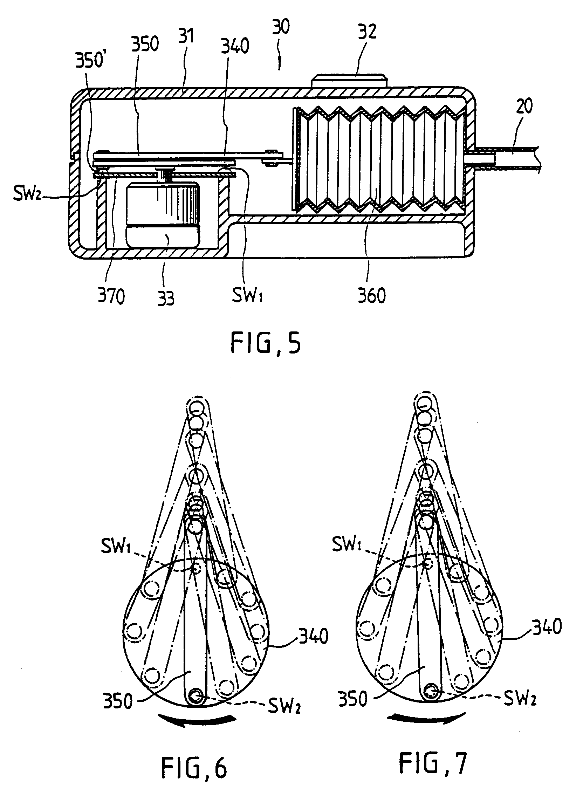

As shown in FIGS 5, 6 and 7, the automated action apparatus 30 comprises a housing 31 which is provided with a plurality of control buttons 32, such as on-off button, reverse control button, speed control button, time control button, ect. Located inside the housing 31 are a frequency variable motor 33 and a rotary wheel 340 driven by the motor 33 which can be controlled by the speed control button. A connection rod 350 is fastened pivotally with the rotary wheel 340 such that the connection rod 350 is connected with an air cell 360. The rotary wheel 340 and the motor 33 are provided therebetween with a control face plate 370 which is provided with a first touch control switch **SW1** and a second touch control switch **SW2**. The first touch control switch **SW1** is used to control the instant acceleration of the motor 33 for increasing the tensile force exerting on the connection rod 350. As the pivoting point 350' has turned to arrive at the second touch control switch **SW2**, the motor 33 is caused to decelerate instantly so as to result in a decrease in the force exerting on the connection rod 350. As a result, the air cell 360 is acted on by the connection rod 350 such that the air pressure inside the eye mask 10 is increased rapidly for treating the myopia. Before the motor 33 is caused to turn counterclockwise, the pressure piece 115 is first removed from the eye mask 10. The counterclockwise rotation of the motor 33 can bring about a decrease in the air pressure inside the eye mask 10 for treating the hyperopia.

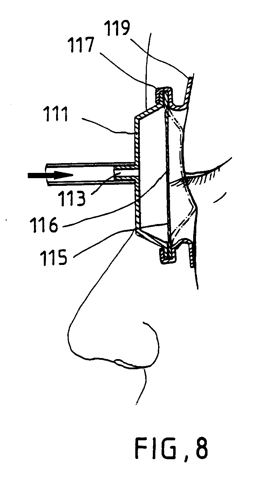

When air is forced from the air cell 360 into the eye mask 10 via the air tube 20, the pressure piece 115 is exerted on by the air pressure to become arcuate in shape. As a result, the buffer piece 116 is forced to press against the eyelid, as shown in FIG. 8. In the meantime, the thicker portion of the pressure piece 115 exerts a pressure on the edge of the eyeball. On the other hand, when the pressure piece 115 is removed from the mask piece 111, the negative pressure in the eye mask 10 can bring about the stretching of the eyeball for treating the hyperopia.

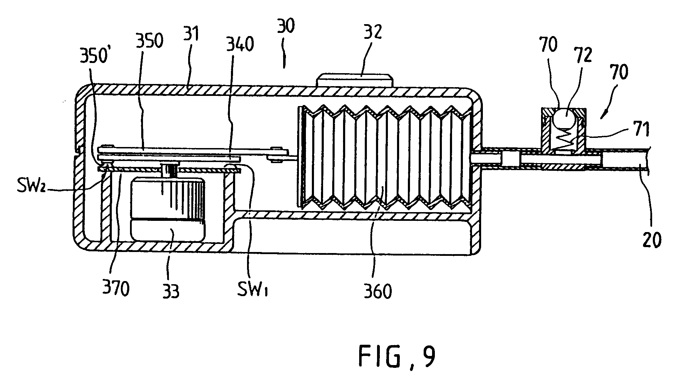

As shown in FIG. 9, another embodiment of the present invention comprises an air tube 20 which is provided with an air valve 70 having therein an elastic piece 71 and a spherical body 72 urged by the elastic piece 71 such that the spherical body 72 is capable of obstructing an air hole 73. When the air cell 360 is pulled backwards, the spherical body 72 is caused to move away from the air hole 73 so as to allow the atmospheric air to enter the air cell 360 via the air hole 73. As a result, an excessive negative pressure in the eye mask 10 can be thus averted.

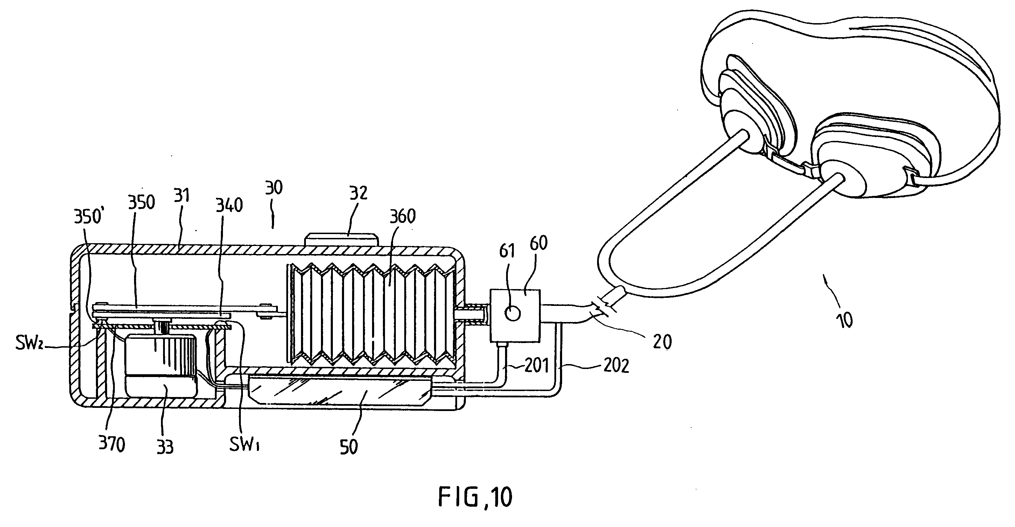

As shown in FIG. 10, the automated action apparatus 30 of the present invention is modified to comprise a control apparatus 50 for regulating an electromagnetic valve 60. When the air cell 360 is acted on by the connection rod 350 to bring about the positive air pressure in the eye mask 10 for treating the myopia, an opening 61 of the electromagnetic valve 60 is sealed off by the control apparatus 50 so as to allow the air to move into the eye mask 10 via the electromagnetic valve 60. On the other hand, when the air cell 360 is pulled backwards by the connection rod 350 to bring about the negative pressure, the control apparatus 50 causes the opening 61 of the electromagnetic valve 60 to open up to allow the entry of the atmospheric air. As a result, the positive air pressure and the negative air pressure can be kept at a precise level by the control apparatus 50 and the electromagnetic valve 60. It must be noted here that the control apparatus 50 is in communication with the electromagnetic valve 60 via an air pipe 201, and that the control apparatus 50 is in communication with the air tube 20 via an air pipe 202. The positive or negative pressure detected by the air pipe 202 is transmitted to the control apparatus 50, which actuates the opening and the closing of the opening 61 of the electromagnetic valve 60 to regulate the positive pressure and the negative pressure in the eye mask 10.

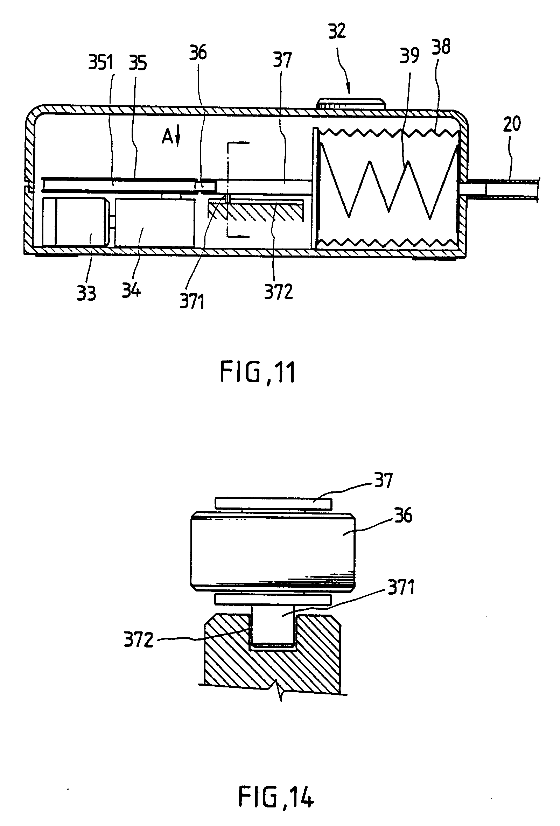

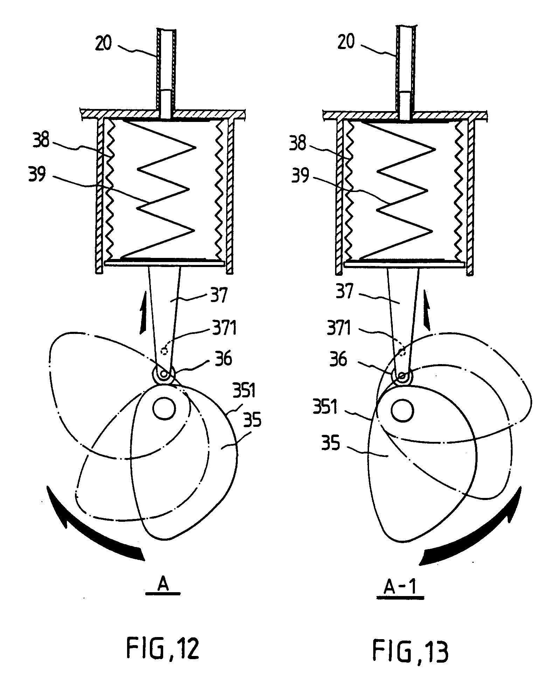

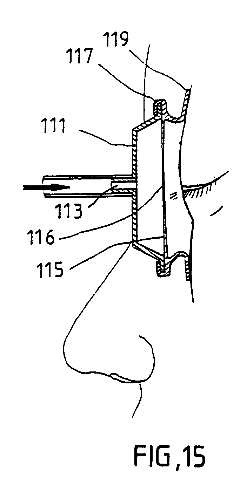

As shown in FIG. 11, the action apparatus 30 is modified such that it is provided with a speed changer 34 which is driven by the motor 33. the rotational speed of the motor 33 can be regulated by the speed control button located on the housing 31 of the action apparatus 30. An eccentric wheel 35 is driven by the speed changer 34 such that the eccentric wheel 35 is caused to rotate clockwise to actuate a roller 36 located on an arcuate surface 351 of the eccentric wheel 35, as shown in FIG. 12. The air cell 38 is thus caused by a push rod 37 to expand or compress to bring about the positive pressure in the eye compress to bring about the positive pressure in the eye mask 10 for treating the myopia. On the other hand, when the motor 33 is caused to turn counterclockwise, the air cell 38 is acted on by the push rod 37 such that the negative pressureis brought about is the eye mask 10 for treating the hyperopia. In order to facilitate the efficient operation of the air cell 38, a spring 39 is disposed in the air cell 38. When the push rod 37 is in motion, the guide column 371 of the push rod 37 is slidably received in a slide slot 372 so as to ensure that the push rod 37 is not forced by the eccentric wheel 35 to move aside. As a result, the air cell 38 is caused to operate with precision, as illustrated in FIG. 14.

As shown in FIG. 15, the mask piece 111 and the frame 117 are joined together at a groove 118. When the air is forced into the eye mask 10 via the air tube 20, the pressure piece 115 is forced by the air pressure to become arcuate in shape, thereby causing the thin buffer piece 116 to press against the eyelid. In the meantime, the thicker portion of the pressure piece 115 is caused to press against the edge of the eyeball for treating the myopia. On the other hand, when the device of the present invention is used for treating the hyperopia, the frame 117 is first removed to allow the mask pieces 111 to press against the face so as to bring about the strectching of the eyeball.

The embodiments of the present invention described above are to be regarded in all respects as being merely illustrative and not restrictive. Accordingly, the present invention may be embodied in other specific forms without deviating from the spirit thereof. The present invention is thereofre to be limited only by the scopes of the following appended claims.

## Claims

1. A vision correcting device comprising an eye mask, an automated action apparatus, and an air tube located between said eye mask and said action apparatus such that said eye mask is caused by said action apparatus to have therein a positive pressure via said air tube for treating myopia, and that said eye mask is caused by said action apparatus to have therein a negative pressure via said air tube for treating hyperopia;
wherein said eye mask is composed of two mask pieces which are opposite in location to each other and are provided respectively with a pressure piece and a frame, said mask pieces further provided respectively with a projection having therein a through hole, said mask pieces still further provided respectively with a rim similar in shape to said pressure piece, said pressure piece having a buffer portion, said frame of said mask pieces provided with a groove and a face frame; and
wherein said action apparatus comprises a housing which is provided thereon with a plurality of control buttons and is provided therein with a variable motor, a rotary wheel ddriven by said motor, and a connection rod fastened pivotally with said rotary wheel such that said connection rod is connected with an air cell capable of being expanded and compressed by said connection rod, said rotary wheel and said motor provided therebetween with a control face plate having a first touch control switch and a second touch control switch, said first touch control switch being used to control an acceleration of said motor to actuate said connection rod to act on said air cell such that air pressure in said eye mask is increased rapidly for treating myopia; and
wherein said pressure piece is first removed from said mask pieces of said eye mask before said motor is caused to turn counterclockwise so as to bring about a decrease in air pressure in said eye mask for treating hyperopia.

2. The vision correcting device as defined in claim 1, wherein said ressure piece is provided with buffer portion and a thick portion and is capable of being expanded by air pressure to become arcuate in shape such that said buffer portion is caused to press against an eyelid, and that said thick portion of said pressure piece is caused to press against the fringe of an eyeball.

3. The vision correcting device as defined in claim 1, wherein said air tube is provided with an air valve having therein an elastic member and a spherical body capable of being urged by said elastic member to seal off an air hole of said air valve, said spherical body further being capable of moving away from said air hole to allow atmospheric air to enter said air cell so as to control air pressure in said eye mask with precision.

4. The vision correcting device as defined in claim 1, wherein said action apparatus further comprises a control device and an electromagnetic valve connected with said control device by an air duct, said control device capable of regulating the passage of air through said electromagnetic valve at such time when said air cell is acted on by said connection rod to bring about a positive pressure in said eye mask for treating myopia; and wherein said control device is capable of regulating the entry of atmospheric air into said electromagnetic valve at such time when said air cell is acted on by said connection rod to bring about a negative pressure in said eye mask for treating hyperopia.

5. The vision correcting device as defined in claim 1, wherein said action apparatus further comprises a speed changing mechanism driven by said motor, and an eccentric wheel actuated by said speed changing mechanism such that an arcuate surface of said eccentric wheel is in contact with an air cell to cause said air cell to expand and compress so as to bring about a positive air pressure and a negative air pressure in said eye mask.
